# EUROPÄISCHE PATENTANMELDUNG

(11) **EP 4 599 863 A1**
(43) Veröffentlichungstag der Anmeldung: **13.08.2025**
(21) Anmeldenummer: 24157175.1
(22) Anmeldetag: 12.02.2024
(51) Int. Cl.: A61M 5/14, A61M 5/142, A61M 39/00, A61M 39/08

(54) **MEDIZINISCHE VORRICHTUNG**

(71) Anmelder: B. Braun Melsungen AG, 34212 Melsungen (DE)
(72) Erfinder: BARTSCH, Stefan, 36286 Neuenstein (DE); ERLEN, Christoph, 34132 Kassel (DE); KELLERMANN, Lukas, 34212 Melsungen (DE)
(74) Vertreter: Tergau & Walkenhorst Intellectual Property GmbH

(57) **Zusammenfassung**

Medizinische Vorrichtung (100), insbesondere eine medizinische Pumpe, umfassend eine Aufnahme oder einen Anschluss (101), eingerichtet zum Einsetzen eines medizinischen Zubehörartikels (200), insbesondere eines medizinischen Einmalartikels, umfassend eine oder verbindbar mit einer Schlauchleitung (201), umfassend
eine Illuminationsquelle (300) zum Illuminieren des eines Teiles einer vom Zubehörartikel (200) umfassten und/oder angeschlossenen Schlauchleitung (201) in einem Sichtbereich (x).

## Beschreibung

### GEBIET DER ERFINDUNG

Die vorliegende Erfindung umfasst eine medizinische Vorrichtung, einen medizinischen Zubehörartikel, ein medizinisches System und ein medizinisches Verfahren zur optimierten Bedienung einer medizinischen Vorrichtung.

### HINTERGRUND DER ERFINDUNG

Im Stand der Technik sind medizinische Vorrichtungen, insbesondere volumetrische Pumpen oder Spritzenpumpen bekannt, die dazu dienen in der Therapie eines Patienten eingesetzt zu werden und insbesondere Medikamente an den Patienten automatisiert zu verabreichen. Dabei werden medizinische Zubehörartikel, insbesondere medizinische Einmalartikel, wie beispielsweise Spritzen oder an Infusionsbeutel angeschlossene Infusionsschläuche, in die medizinische Vorrichtung eingebracht, die sodann das in dem Zubehörartikel, beispielsweise einer Spritze für eine Spritzenpumpe oder einem Infusionseinmalartikel, z.B. Infusionsbeutel, enthaltene Medikament oder medizinische Lösung kontrolliert an den Patienten befördert. Dabei wird insbesondere das in der medizinischen Vorrichtung vorhandene Aktuator- und/oder Pumpensystem verwendet, das durch eine entsprechende Steuereinrichtung angesteuert werden kann.

Beispielsweise kann ein Medikament oder eine andere medizinische Lösung, wie beispielsweise Kochsalzlösung, in einer medizinischen Spritze oder einem Infusionsbeutel bereitgestellt werden. Diese Zubehörartikel mit beinhaltendem Medikament oder medizinischer Lösung können dann in die medizinische Vorrichtung eingebracht werden, und der Aktuator, der die Spritze oder die Pumpe die den Infusionsschlauch betätigt, werden sodann so angesteuert, dass die gewünschte Menge Lösung dem Patienten verabreicht wird.

Es sind eine Vielzahl solcher medizinischen Vorrichtungen, sowie zugehöriger Zubehörartikel im Stand der Technik bekannt.

Es besteht jedoch im Allgemeinen die Herausforderung, dass im medizinischen Alltag und gegebenenfalls im Hausgebrauch, das richtige Medikament oder die richtige medizinische Lösung, über den gewünschten Zugang, in der gewünschten Menge und Zeit beim Einsatz mehrerer medizinischer Vorrichtungen an den Patienten gelangt, und das medizinische System, umfassend die medizinische Vorrichtung und den medizinischen Zubehörartikel so eingerichtet sein soll, dass eine Verwechslung oder anderweitige versehentliche Falschbedienung möglichst ausgeschlossen wird.

In der Praxis wählt zumeist klinisches Personal einen mit medizinischer Lösung und gegebenenfalls Medikament vorbestückten Zubehörartikel, oder bestückt diesen selbst mit Lösung und gegebenenfalls Medikament, und setzt sodann den bestückten Zubehörartikel in die medizinische Vorrichtung ein. Gegebenenfalls kann an einer grafischen Benutzeroberfläche der medizinischen Vorrichtung der ausgewählte Zubehörartikel ausgewählt und bestätigt werden. Modernere Systeme verfügen über eine entsprechende Netzwerkschnittstelle und kommunizieren gegebenenfalls mit einer externen Steuereinheit.

Dies ist von besonderer Relevanz, wenn medizinische Systeme, beispielsweise im intensivmedizinischen Bereich, eine Vielzahl von medizinischen Vorrichtungen umfassen, um den Patienten zur selben Zeit eine Vielzahl von verschiedenen Medikamenten und Lösungen, gegebenenfalls über eine Vielzahl von Zugängen bereitzustellen. Dann können hierdurch eine Vielzahl von medizinischen Schläuchen den Patienten umgeben und die Sicherheit vor Fehlern beim Anschluss durch medizinisches Personal kann beeinträchtigt sein.

Wird das falsche Medikament verabreicht, oder eine falsche Förderleistung der medizinischen Vorrichtung gewählt, kann dies zu fatalen Folgen für den Patienten führen. Insbesondere kann die Gabe von Medikamenten in der falschen Patientenzugang unmittelbar hochkritisch sein, oder - insbesondere bei eingestellten geringen Förderraten - kann ein etwaiger vorhandener Alarm erst spät ausgelöst werden, der eine zu geringe Gabe an Medikament oder medizinischer Lösung detektieren würde. Auch die Vermischung von ggf. nicht miteinander kompatiblen Medikamenten und/oder Lösungen kann durch eine fehlende Übersicht über den Leitungsverlauf verschiedener Zubehörartikel und medizinischer Vorrichtungen vorkommen.

Gegenwärtig wird die Identifizierung der geeigneten Zubehörartikel von Hand durchgeführt und insbesondere durch eine visuelle und manuelle Verfolgung des Schlauchs von der medizinischen Vorrichtung hin zum Patientenzugang. Dabei sind im Stand der Technik verschiedene Lösungen bekannt, die insbesondere eine Markierung der Zubehörartikel vorsehen, um einer Verwechslung beim Bestücken der medizinischen Vorrichtung mit dem Zubehörartikel möglichst entgegenzuwirken. Beispielsweise offenbart Dokument, WO 23280455 A1 einen Einwegmedizin-artikel, insbesondere einen medizinischen Schlauch oder eine medizinische Gleit-schelle, zur Verwendung an oder in einem medizinischen Gerät, wobei der Einwegmedizinartikel mindestens eine integrierte Markierung als Identifikationsmerk-mal umfasst. Dabei ist die mindestens eine Markierung an einem vordefinierten Abschnitt des Einwegartikels mit bspw. einer Farbe vorgesehen.

Dokument EP 4205779 A1 offenbart eine Infusionspumpe zur Erkennung der Farbe einer Schlauchklemme mit IR-Markern.

Jedoch führen die im Stand der Technik bekannten Lösungsansätze zu erhöhtem Aufwand für die Pflegekräfte, da diese die Leitungsführung mühsam manuell nachvollziehen müssen. Selbst bei der Verwendung von Infusionsleitungen mit Farbkennzeichnung kann nur bedingt Übersichtlichkeit geschaffen werden. Insbesondere, da keine ausreichend große Anzahl unterschiedlicher Farben zur Verfügung steht, um alle Leitungen in einem größeren medizinischen Infusionssystem unterscheidbar zu machen.

Es bleibt dabei insbesondere auch mit Hinblick auf den Einsatz in einem dunklen Umfeld oder weitestgehend unbeleuchteten Umfeld problematisch, Fehler beim Anschluss und in der Zuordnung von Zubehörartikel und medizinischer Vorrichtung zu vermeiden.

Das Dokument WO 2013 074717 A1 offenbart einen beleuchteten Schlauchsatz. Bei den Zubehörartikeln handelt es sich jedoch zumeist um Einmalartikel, die nach Gebrauch entsorgt werden.

### ZUSAMMENFASSUNG DER ERFINDUNG

Es ist eine der Erfindung zu Grunde liegende Aufgabe, die oben genannten und weitere Nachteile zu überwinden. Dabei ist es eine weitere der Erfindung zu Grunde liegende Aufgabe, die Sicherheit beim bestimmungsgemäßen Anschluss von medizinischen Lösungen und ggf. Medikamente umfassenden Zubehörartikeln in eine medizinische Vorrichtung zu erhöhen und insbesondere sicherzustellen, dass der jeweilige Zubehörartikel der bestimmungsgemäß richtigen medizinischen Vorrichtung zugeordnet wird. Darüber hinaus ist es eine der Erfindung zu Grunde liegende Aufgabe, die bestimmungsgemäße Einstellung der medizinischen Vorrichtung, insbesondere mit Hinblick auf die Abgabe von medizinischer Lösung und ggf. Medikament, das im Zubehörartikel umfasst ist, an den Patienten, zu erleichtern.

Diese und andere Probleme werden durch den Gegenstand der beigefügten unabhängigen Ansprüche gelöst.

### AUSFÜHRLICHE BESCHREIBUNG DER ERFINDUNG

Die oben genannten Aufgaben werden erfindungsgemäß gelöst, durch eine medizinische Vorrichtung, durch einen medizinischen Zubehörartikel, ein medizinisches System, ein medizinisches Verfahren und ein computerlesbares Speichermedium gemäß den beigefügten Ansprüchen.

Bevorzugte Ausführungsformen können den abhängigen Ansprüchen entnommen werden und darüber hinaus aus der folgenden Beschreibung, insbesondere unter Einbeziehung verschiedener Ausführungsformen, wie sie in den beigefügten Ansprüchen abgedeckt und beschrieben sind.

Der Fachmann wird verstehen, dass jede Ausführungsform, die in der folgenden Beschreibung erläutert wird, durch den Gegenstand der beigefügten Ansprüche abgedeckt und umfasst ist. Die in Verbindung mit der Erfindung beschriebenen Ausführungsformen, Merkmale und Kombinationen von Merkmalen, sowie die in den beigefügten Ansprüchen angegebenen Kombinationen von Merkmalen, aber auch jede in Verbindung mit den Ausführungsformen erwähnte und beschriebene Kombination von Merkmalen, gelten zumindest jedoch als durch den Fachmann ableitbar.

Insbesondere kann jedes Merkmal und jede Kombination von Merkmalen in den hier beschriebenen Ausführungsformen beispielsweise in einer anderen Kombination beansprucht werden, insbesondere in einer anderen Anspruchskategorie, da der Fachmann erkennen wird, dass jede einzelne Kombination der hier genannten Merkmale dazu geeignet ist, zur Lösung des zugrunde liegenden Problems beizutragen.

Weiterhin kann jedes Merkmal und jede Kombination von Merkmalen in den Ansprüchen und in der nachstehenden Beschreibung unabhängig von dem jeweiligen beanspruchten Gegenstand, unabhängig von Anspruchsabhängigkeiten und Rückverweisen sowie unabhängig von der Anspruchskategorie, in der das Merkmal beansprucht wird, verwendet und beansprucht werden. Beispielsweise kann in einer beliebigen Kombination, die aus einem oder mehreren Ansprüchen ausgewählt ist, eine oder mehrere der unten beschriebenen Ausführungsformen und/oder der beigefügten Figuren vorgesehen werden.

Innerhalb der vorliegenden Anwendung beziehen sich Begriffe wie "seitlich", "hinten", "vorne", "oben", "unten", "gegenüber", "innen", "außen" oder ähnliche, wie hier verwendet, auf die Position eines ersten Objekts relativ zu einem anderen Objekt. Vorzugsweise beziehen sie sich auf die relative Position eines jeweiligen Teils oder Objekts in Bezug auf seine Position, wenn es vollständig für seinen beabsichtigten Gebrauch montiert ist.

Es wird sofort von einer fachkundigen Person erkannt, dass ein Merkmal, eine Ausführungsform, eine Wirkung oder ein Vorteil, die hier in Verbindung mit der erfinderischen medizinischen Vorrichtung beschrieben sind, auch ein Merkmal, eine Ausführungsform, eine Wirkung oder ein Vorteil des erfinderischen Verfahrens, des erfinderischen Zubehörartikels, des medizinischen Systems und/oder des computerlesbaren Speichermediums sein können, und umgekehrt.

Die der Erfindung zu Grunde liegenden Aufgaben werden in einem ersten Aspekt gelöst durch eine medizinische Vorrichtung, insbesondere eine medizinische Pumpe nach den beigefügten Ansprüchen. Die medizinische Vorrichtung kann insbesondere eine volumetrische Pumpe, wie bspw. eine Infusionspumpe, oder eine Spritzenpumpe, zum Beispiel ein Spritzenpumpe sein.

Die erfindungsgemäße Vorrichtung umfasst eine Aufnahme oder einen Anschluss für einen medizinischen Zubehörartikel. Die oder der Anschluss ist so, eingerichtet, dass der medizinische Zubehörartikel eingesetzt, insbesondere angeschlossen und/oder angekoppelt werden kann. Dabei kann bestimmungsgemäß der gesamte oder nur ein Teil des Zubehörartikels in die medizinische Vorrichtung eingesetzt werden.

Der medizinische Zubehörartikel umfasst eine Schlauchleitung oder ist mit einer solchen Schlauchleitung verbindbar. Der Fachmann kennt derartige medizinische Zubehörartikel und weiß, dass diese in unterschiedlicher Konfiguration im Markt erhältlich und in der Vorrichtung nach der vorliegenden Erfindung einsetzbar sind. Dabei kann ein solcher Zubehörartikel einteilig sein und insbesondere die Schlauchleitung bereits umfassen, oder dazu eingerichtet sein mit einer Schlauchleitung verbindbar zu sein. Die Schlauchleitung kann zum Beispiel an den Zubehörartikel angeschlossen oder aufgesteckt werden.

Die erfindungsgemäße Vorrichtung umfasst weiter eine Illuminationsquelle zum Illuminieren des eines Teiles einer vom Zubehörartikel umfassten und/oder angeschlossenen Schlauchleitung in einem Sichtbereich.

Der Begriff "Sichtbereich" wie hierin verwendet bezieht sich bevorzugt auf die Wahrnehmung eines Benutzers, insbesondere eines medizinischen Personals, das die medizinische Vorrichtung bestimmungsgemäß verwendet oder den bestimmungsgemäßen Gebrauch kontrolliert. Dabei ist der Sichtbereich bevorzugt ein Bereich, der im Zustand des bestimmungsgemäßen Gebrauchs der Vorrichtung, also bevorzugt mit eingesetztem Einmalartikel, für den Benutzer sichtbar ist. Mit anderen Worten ist ein Sichtbereich für einen Benutzer auch dann sichtbar, wenn die Aufnahme oder der Anschluss zum Einsetzen des Zubehörartikels, beispiels-weise durch eine Schutz- oder Verschlussklappe, verschossen ist.

Die vorliegende Erfindung erlaubt dem Benutzer, insbesondere einem medizinischen Personal, auf vorteilhafte Weise eine sichere und schnelle Überprüfung mittels einfacher optischer Inspektion, dass des Anschlusses von medizinischen Lösungen und ggf. Medikamenten, die im Zubehörartikel umfasst sind, in einer medizinischen Vorrichtung bestimmungsgemäß sind. Insbesondere kann leicht sichergestellt werden, dass der jeweilige Zubehörartikel der bestimmungsgemäß richtigen medizinischen Vorrichtung zugeordnet wird.

Durch die Illuminationsquelle wird nämlich vorteilhaft ein Teil einer Schlauchleitung, die entweder angeschlossen an oder Teil eines Zubehörartikels ist, in einem für den Benutzer zugänglichen - sichtbaren - Sichtbereich illuminiert. Hierdurch kann insbesondere eine leichtere Erkennung und Zuordnung eines im Zubehörartikel enthaltenen Medikaments oder medizinischen Lösung, bzw. auch der damit am Patienten geplanten Therapie, erhalten werden.

Besonders vorteilhaft wird der Verlauf und/oder Anschluss von Schlauchleitungen, insbesondere Infusionsleitungen, mittels eines Illuminationssignals, insbesondere Lichtsignals, besser nachvollziehbar.

Dabei sorgt die Illuminationsquelle, zum Beispiel eine Lichtquelle, wie das Licht einer Lampe, dafür, dass die Schlauchleitung in bestimmten Bereichen, besonders im Sichtfeld des Benutzers, beleuchtet, also illuminiert, wird. Das Licht breitet sich beispielsweise in einer Infusionsleitung aus und kann durch Streuung oder ähnliche physikalische Effekte kontinuierlich oder an speziell vorgesehenen Stellen austreten. Auf diese Weise ist es möglich, den Verlauf der einzelnen Schlauchleitungen durch die Illuminationssignale auf einfache Weise nachzuverfolgen.

In einer vorteilhaften und bevorzugten Ausführungsform der medizinischen Vorrichtung nach der vorliegenden Erfindung ist die Illuminationsquelle eine Lichtquelle. Die Lichtquelle ist bevorzugterweise ausgewählt aus der Gruppe umfassend LED und Laser. Hierdurch wird insbesondere vorteilhaft sichergestellt, dass die ausgewählte Lichtquelle, insbesondere aus der Gruppe von LEDs und Lasern, der medizinischen Vorrichtung nach der vorliegenden Erfindung ermöglicht, eine präzise und effektive Illumination bereitzustellen. LEDs und Laser zeichnen sich durch ihre präzise Steuerbarkeit, relativ geringe Kosten, geringen Energieverbrauch und ihre Fähigkeit zur gezielten Ausrichtung des Lichts aus, was zu einer verbesserten Leistung und Anpassungsfähigkeit der Vorrichtung führt.

Alternativ können aber auch andere Illuminationsquellen erfindungsgemäß eingesetzt werden. Beispielsweise kann anstelle von Licht, ein elektrisches Wechselfeld verwendet und in die Schlauchleitung eingekoppelt werden. Das Illuminationssignal kann dann auf Basis von Elektro-Lumineszenz Effekten erzeugt werden. Weiterhin können auch elektrische Ströme als Illuminationsmittel in die Infusionsleitung eingekoppelt werden, die von einer Stromquelle als Illuminationsquelle erzeugt werden. Das Lichtsignal wird dann bspw. mittels Emission an Halbleiterübergängen erzeugt. Hierzu kann die erfindungsgemäße Vorrichtung eine geeignete Illuminationsquelle, zum Beispiel organische Leuchtdioden, umfassen.

In einer vorteilhaften und bevorzugten Ausführungsform der medizinischen Vorrichtung nach der vorliegenden Erfindung umfasst die medizinische Vorrichtung eine Führung für die Schlauchleitung. Eine solche Führung ist in den meisten üblichen medizinischen Vorrichtungen des Standes der Technik vorhanden, um den Zubehörartikel, bzw. die umfasste und/oder angeschlossene Schlauchleitung, bestimmungsgemäß zu fixieren. Die Illuminationsquelle kann vorteilhaft besonders einfach im Bereich der Führung der Schlauchleitung angebracht werden. In einer Ausführungsform ist eine erste Illuminationsquelle im Bereich der Führung der Schlauchleitung innerhalb der medizinischen Vorrichtung, also in einem Bereich des Einlegens des Artikels, so angeordnet, dass der Sichtbereich eines Austritts der Schlauchleitung aus der medizinischen Vorrichtung beleuchtet wird, um die Schlauchleitung außerhalb der medizinischen Vorrichtung zu illuminieren. Zusätzlich oder alternativ, ist eine zweite Illuminationsquelle im Bereich der Führung der Schlauchleitung innerhalb der medizinischen Vorrichtung, in einem Bereich des Einlegens des Artikels, so angeordnet, dass der Sichtbereich eines Eintritts der Schlauchleitung in die medizinische Vorrichtung beleuchtet wird, um die Schlauchleitung außerhalb der medizinischen Vorrichtung zu illuminieren.

Die Begriffe "Austritt" und "Eintritt", wie hierin verwendet, bezeichnen bevorzugt Bereiche der medizinischen Vorrichtung, die bezogen sind auf eine bestimmungsgemäße Flussrichtung (F) eines in dem medizinischen Zubehörartikel bereitgestellten Medikaments oder einer medizinischen Lösung. Mit anderen Worten ist der Austritt einer Schlauchleitung in einer medizinischen Vorrichtung jener Bereich, an dem in dem Schlauchteil ein in dem medizinischen Zubehörartikel bereitgestellten Medikaments oder einer medizinischen Lösung austritt oder ausfließt. Entsprechend ist der Eintritt einer Schlauchleitung in einer medizinischen Vorrichtung jener Bereich, an dem in dem Schlauchteil ein in dem medizinischen Zubehörartikel bereitgestellten Medikaments oder einer medizinischen Lösung eintritt oder einfließt.

Zur besseren Orientierung kann in einer Ausführungsform die medizinische Vorrichtung Illuminationskonturen aufweisen. Derartige Illuminationskonturen können vorteilhaft das Einkoppeln eines Illuminationssignals in die Schlauchleitung erleichtern. Gleichzeitig oder alternativ können Illuminationskonturen als Illuminationsleitkonturen ausgebildet sein, die vorteilhaft dem Leiten der Illumination zu einem Einkoppelpunkt des Illuminationssignals in die Schlauchleitung dienen.

In einer vorteilhaften und bevorzugten Ausführungsform der medizinischen Vorrichtung nach der vorliegenden Erfindung umfasst die Illuminationsquelle eine oder mehrere Lichtquellen. Bevorzugt sind die eine oder die mehreren Lichtquellen eingerichtet, Licht verschiedener Wellenlängen zu erzeugen. Mit anderen Worten kann die Lichtquelle verschiedenfarbiges Licht erzeugen. Insbesondere kann die Lichtquelle hierzu verschiedenfarbige Lichtquellen umfassen. Insbesondere kann die Lichtquelle verschiedenfarbige LEDs, RGB-LEDs, oder ähnliches umfassen. Diese Lichtquelle, insbesondere LEDs können vorteilhaft auf einer einzelnen oder mehreren Leiterplatten angeordnet sein. Vorteilhaft kann in derartigen Anordnungen die Lichtquelle unterschiedliche Farben ausgeben, die sodann jeweils im Zusammenhang mit der Art der durch die Schlauchleitung verabreichten Medikation, bezogen auf Inhalt und/oder Therapieschema, stehen können. Alternativ kann auch die Auswahl der Illumination, insb. der Farbe, auf der Erkennung des Einmalartikels bspw. mittels eines Sensors einer Detektionseinheit zur Detektion des Zubehörartikels und/oder zur Klassifizierung und/oder Einordnung des Zubehörartikels erfolgen. Mit anderen Worten, können je nach erkanntem Zubehörartikel zum Beispiel unterschiedliche Farben ausgeben werden, die jeweils im Zusammenhang mit der Art des Zubehörartikels, oder auch eines bspw. daran angebrachten Markers stehen können.

Somit lässt sich leicht anhand der Farbe die Medikation, bezogen auf Inhalt und/oder Therapieschema, der medizinischen Vorrichtung zuweisen und eine Verwechslungsgefahr oder die Wahrscheinlichkeit für einen Falschanschluss wird vorteilhaft herabgesetzt.

Vorteilhaft kann die Beleuchtungsfarbe den Einstellungen in einer Medikamentendatenbank zugewiesen sein. Dies kann in einem erfindungsgemäßen System fabrikationsseitig vorgesehen sein und/oder durch einen Benutzer einstellbar sein.

Dabei kann eine gewählte oder vorbestimmte Farbe einer Medikamentengruppe zugewiesen sein.

Zur besseren Zuordnung kann eine medizinische Vorrichtung nach der vorliegenden Erfindung ein Anzeigeelement aufweisen, bspw. auf einem Benutzerinterface, aufweisen, zum Beispiel eine separate Leuchtanzeige, das ganze Display oder Teile des auf dem Display angezeigten Benutzerinterface, das eingerichtet ist, ein Signal bereitzustellen, das in der gleichen Farbe wie der Zubehörartikel, ein Marker des Zubehörartikels und/oder das Illuminationssignal gestaltet ist.

In einer weiteren Ausführungsform kann die medizinische Vorrichtung nach der vorliegenden Erfindung so eingerichtet sein, dass ein Farb-Muster aus wechselnden Farben als Illuminationssignal bereitgestellt werden kann, um deren Zusammengehörigkeit zu signalisieren.

In einer vorteilhaften und bevorzugten Ausführungsform der medizinischen Vorrichtung nach der vorliegenden Erfindung umfasst die medizinische Vorrichtung eine Detektionseinheit mit einem Sensor, insbesondere einen Berührungssensor. Ein solcher Sensor kann eingerichtet sein eine Berührung durch den Anwender zu detektieren. Beispielsweise kann der Sensor so gestaltet sein, dass über elektrische Effekte, zum Beispiel eine Potentialveränderung, das Auftreten, beziehungsweise die Veränderung von Ableitströmen oder optische Effekte, zum Beispiel eine Rückreflektionen des Illuminationssignals, eine Berührung der Schlauchleitung detektiert werden kann. Ein solches Berührungssignal kann als Steuersignal, bspw. für ein An- oder Abschalten der Illumination durch die medizinische Vorrichtung, insbesondere durch eine Steuereinheit der medizinischen Vorrichtung, verwendet werden. Darüber hinaus kann eine derartige Sensorinformation der Detektion von Berührungen auch an die Steuerungseinheit der medizinischen Vorrichtung, bspw. der volumetrischen Pumpe, weitergeben werden. Zusätzlich oder alternativ kann der Sensor auch zur Detektion und insbesondere Klassifizierung oder Einordnung eines Zubehörartikels dienen. Dies erlaubt insbesondere, anhand des detektierten und damit identifizierten Zubehörartikels, den Einmalartikel und/oder einen Teil einer vom Zubehörartikel umfassten und/oder angeschlossenen Schlauchleitung in einem Sichtbereich zu illuminieren.

Die Steuereinheit einer medizinischen Vorrichtung nach der vorliegenden Erfindung kann dazu eingerichtet sein, Parameter der medizinischen Vorrichtung basierend auf derartigen Steuersignalen zu verändern. Mit anderen Worten sind dann verschiedene Parameter der medizinischen Vorrichtung durch Berührung der Schlauchleitung einstellbar. Ausgehend von einer Detektion der Berührung des Zubehörartikels durch den Anwender, kann dann bspw. die Beleuchtung, oder weitere, andere Parameter und Steuerabläufe in der medizinischen Vorrichtung kontrolliert werden. So kann sich eine Pflegekraft zum Beispiel schneller in einem Leitungsgewirr von mehreren Infusionsleitungen orientieren und den Leitungsverlauf einer ausgewählten Leitung nachverfolgen, wenn die von der Pflegekraft berührte Schlauchleitung illuminiert und dadurch gegenüber den anderen Leitungen erkennbar wird.

In einer vorteilhaften und bevorzugten Ausführungsform der medizinischen Vorrichtung nach der vorliegenden Erfindung umfasst die medizinische Vorrichtung eine Lichtweiche, insbesondere ein Prisma. Über eine derartige Lichtweiche kann das Illuminationssignal der Illuminationsquelle vorteilhaft gelenkt und/oder fokussiert werden, und bspw. gebündelt werden, bzw. punktförmig die Schlauchleitung illuminieren. Die Illuminationsquelle kann durch eine derartige Lichtweiche ebenfalls, das Illuminationssignal in die Schlauchleitung, bzw. das Schlauchleitungsmaterial, einkoppeln. Hierzu sind bestimmungsgemäße Schlauchleitungen vorteilhafterweise zumeist durchsichtig oder durchscheinend, zumeist aus farblosem Kunststoff gefertigt. Die Illuminationsquelle, insbesondere Lichtquelle, koppelt so vorteilhaft das Illuminationssignal, mittels der Lichtweiche als in die transparente Infusionsleitung ein. Dies kann bspw. vorteilhaft über einen typischen, zylindrischen Durchmesser des Mantels einer solchen Schlauchleitung geschehen. Hierzu kann eine erfindungsgemäße Vorrichtung eine Lichtweiche, insbesondere ein Prisma, umfassen, das einen für die Lichteinspeisung günstigen Einfallwinkel des Lichts relativ zu der Schlauchleitung aufweist.

Eine medizinische Vorrichtung nach der vorliegenden Erfindung kann weiterhin ein Betätigungsmittel zum, bevorzugt manuellen, Ein- und/oder Ausschalten der Illuminationsquelle umfassen. Im einfachsten Fall umfasst die Vorrichtung einen An-/Aus-Knopf, mittels dem sich die Illumination, beispielsweise durch Unterbrechung der Stromversorgung zur Illuminationsquelle, an und ausschalten lässt. Somit kann der Anwender ein Illuminationssignal aktivieren oder abschalten. Zum Illuminationssignal aktivieren oder abschalten kann in einer weiteren Ausführungsform die erfindungsgemäße medizinische Vorrichtung eine Benutzeroberfläche umfassen, bspw. einen Touchscreen, oder bestimmungsgemäße andere Betätigungsmittel, oder andere bereits für den Betrieb der Pumpe notwendige Bedienelemente, zum Beispiel einen Spritzenbügel, einen Öffnungsmechanismus für eine Frontklappe, eine Legekontur für den Zubehörartikels, usw.

Eine medizinische Vorrichtung nach der vorliegenden Erfindung kann weiter eine Detektionseinheit zur Detektion eines Zubehörartikels umfassen. Insbesondere kann eine derartige Detektionsseinheit einen Sensor aufweisen, beispielsweise einen Pumpensensor, der eingerichtet ist zur Detektion zumindest eines, insbesondere auf die Detektionseinheit abgestimmten, Markers des medizinischen Zubehör-artikels. Ein derartiger Marker kann am Zubehörteil durch einen Benutzer angebracht sein und lösbar oder unlösbar mit dem Zubehörteil verbunden werden, oder als integrierter Marker werksseitig bereitgestellt sein. Ein derartiger Marker ist bereit-gestellt als Erkennungsmerkmal des eingesetzten oder angeschlossenen Zubehör-artikels. Eine Steuereinheit der medizinischen Vorrichtung kann dafür eingerichtet sein, anhand des detektierten Erkennungsmerkmals den Zubehörartikels zu identifizieren und/oder eine Lage des Zubehörartikels gegenüber der Detektionseinheit zu bestimmen. Ein derartiges Merkmal kann auf unterschiedliche Weise bereitgestellt werden und beispielsweise in farbiges Merkmal, z.B. eine farbige Schlauchklemme sein.

Die der Erfindung zu Grunde liegenden Aufgaben werden in einem zweiten Aspekt gelöst durch einen medizinischer Zubehörartikel. Ein medizinischer Zubehörartikel nach der vorliegenden Erfindung ist insbesondere ein Einmalartikel. Ein medizinischer Zubehörartikel nach der vorliegenden Erfindung weist wenigstens abschnittsweise einen Hohlkörper auf, beispielsweise eine Schlauchleitung oder einen Schlauchleitungsabschnitt. Ein medizinischer Zubehörartikel nach der vorliegenden Erfindung ist eingerichtet zum Einsetzen, Einschließen und/oder Ankoppeln in oder an eine medizinische Vorrichtung, wobei bevorzugt die medizinische Vorrichtung eine medizinische Vorrichtung nach der vorliegenden Erfindung ist.

Der medizinische Zubehörartikel kann insbesondere ein medizinischer Einmalartikel sein, wie beispielsweise eine Spritze, ein medizinischer Schlauch, ein Infusionsbeutel mit Schlauchleitung oder Infusionsbeutel vorgesehen zum Verbinden mit einer Schlauchleitung. Der medizinische Zubehörartikel kann also eine Schlauchleitung umfassen oder mit einer Schlauchleitung verbindbar sein.

In einer vorteilhaften und bevorzugten Ausführungsform des medizinischen Zubehörartikels nach der vorliegenden Erfindung, umfasst der Zubehörartikel einen Lichtwellenleiter und/oder ein Lichtreflektionsmittel. Hiermit kann der Zubehörartikel und insbesondere ein Teil einer vom Zubehörartikel umfassten und/oder angeschlossenen Schlauchleitung in einem Sichtbereich besonders vorteilhaft illuminiert werden. Alternativ kann der medizinischen Zubehörartikels nach der vorliegenden Erfindung ein Lichtleitelement umfassen, das an die Schlauchleitung angefügt und/oder angearbeitet ist. Dieses ist bevorzugt so ausgestaltet, dass eine vorteilhafte Einkopplung des Illuminationssignals, insbesondere des Lichts, erreicht wird.

In einer vorteilhaften und bevorzugten Ausführungsform des medizinischen Zubehörartikels nach der vorliegenden Erfindung, umfasst der medizinische Zubehörartikel eine Illuminationsquelle. Ein Zubehörartikel mit einer solchen, eigenen Illuminationsquelle zusätzlich oder alternativ zu einer Illuminationsquelle einer medizinischen Vorrichtung nach dem ersten Aspekt der Erfindung, dient vorteilhaft zum Illuminieren eines Teiles einer vom Zubehörartikel umfassten und/oder angeschlossenen Schlauchleitung in einem Sichtbereich. Mit anderen Worten kann auch der Zubehörartikel selbst eine Illuminationsquelle aufweisen und sich selbst illuminieren. Hier ist unmittelbar ersichtlich, dass in einem erfindungsgemäßen System besonders vorteilhaft die medizinische Vorrichtung und der Zubehörartikel jeweils über eine Illuminationsquelle verfügt. So kann, nachfolgend einem Einlegen des Zubehörartikels in eine medizinische Vorrichtung, bspw. ein Farbabgleich der beiden Illuminationen eine korrekte Zuordnung deutlich erleichtern. Insoweit wird der Fachmann unmittelbar erkennen, dass die Illuminationsquelle des erfindungsgemäßen Zubehörartikels die gleichen Eigenschaften und Merkmale aufweisen kann, wie eine Illuminationsquelle der erfindungsgemäßen Vorrichtung nach dem ersten Aspekt der Erfindung.

Wenn also bspw. eine Illuminationsquelle der erfindungsgemäßen Vorrichtung hierin als Lichtquelle, bevorzugt LEDs, Laser oder dergleichen beschrieben ist, so sind diese Merkmale ebenfalls mögliche Ausführungsformen der Illuminationsquelle des erfindungsgemäßen Zubehörartikels. Insbesondere kann das Illumineszenzsignal auf Basis von Elektrolumineszenz-Effekten erzeugt werden. Auch können elektrische Ströme in die Schlauchleitung eingekoppelt werden. Das Illumineszenzsignal kann bspw. mittels Emission an Halbleiterübergängen erzeugt werden, zum Beispiel mit organischen Leuchtdioden.

In einer Ausführungsform umfasst der medizinische Zubehörartikel eine Lichtleitung entlang eines Mantelteils des Zubehörartikels. Der Illuminationseffekt kann so vorteilhaft entlang des Mantelteils, insbesondere entlang der Schlauchleitung erzeugt werden. Hierzu kann der Zubehörartikel, insbesondere in einem derartigen Mantelteil, hinsichtlich des gewählten Materials, zum Beispiel hinsichtlich von Fluoreszenzeigenschaften, bereitgestellt sein.

Die der Erfindung zu Grunde liegenden Aufgaben werden in einem dritten Aspekt gelöst durch ein medizinisches System umfassend wenigstens eine medizinische Vorrichtung, bevorzugt nach dem ersten Aspekt der Erfindung, und wenigstens ein Zubehörteil, bevorzugt nach dem zweiten Aspekt der Erfindung. Das erfindungsgemäße System umfasst weiter eine Speichereinheit mit einer Medikamentendatenbank, in welcher Daten zu Medikamenten hinterlegt sind. Vorzugsweise ist in der Speichereinheit eine Zubehörartikel-Datenbank gespeichert, in welcher Daten zu zumindest zwei unterschiedlichen Zubehörartikel hinterlegt sind. Die Steuereinheit des erfindungsgemäßen Systems ist dafür vorgesehen und angepasst, anhand des detektierten und damit identifizierten Zubehörartikels, einen Teil einer vom Zubehörartikel umfassten und/oder angeschlossenen Schlauchleitung in einem Sichtbereich zu Illuminieren. Mit anderen Worten kann abhängig bzw. bezogen auf den Einmalartikel ein Teil einer vom Zubehörartikel umfassten und/oder angeschlossenen Schlauchleitung in einem Sichtbereich illuminiert werden.

Besonders vorteilhaft ist das erfindungsgemäße System auch in der Lage eine Medikamentenauswahlmöglichkeit in der Medikamentendatenbank zu filtern und/oder zu begrenzen und/oder eine Auswahlmöglichkeit eines weiteren Zubehörartikels zu filtern und/oder zu begrenzen. Das erfindungsgemäße System kann auf die Daten hinterlegter Medikamente, aber auch auf hinterlegte Therapieschemata für einzelne Medikamente und/oder medizinische Lösungen, oder auch auf hinterlegte Therapieschemata für eine Kombination verschiedener Medikamente eingerichtet sein. Somit sind auch, wenn das System eine Mehrzahl an medizinischen Vorrichtungen und/oder medizinischen Zubehörartikel umfasst, eine eindeutige Zuordnung von Medikamenten- und/oder Therapiedaten der Medikamentendatenbank zu den medizinischen Vorrichtungen und/oder medizinischen Zubehörartikel möglich.

Insbesondere in Ausführungsformen, in denen das erfindungsgemäße System eine medizinische Vorrichtung nach dem ersten Aspekt der Erfindung umfasst und/oder in Ausführungsformen, in denen das erfindungsgemäße System einen medizinischen Zubehörartikel nach dem zweiten Aspekt der Erfindung umfasst, kann die Steuereinheit des erfindungsgemäßen Systems anhand des detektierten und damit identifizierten Zubehörartikels, einen Teil einer vom Zubehörartikel umfassten und/oder angeschlossenen Schlauchleitung in einem Sichtbereich zu illuminieren. Dies kann vorteilhaft durch eine Illuminationsquelle der medizinischen Vorrichtung oder wahlweise oder zusätzlich durch eine Illuminationsquelle des Zubehörartikels erreicht werden. Damit wird mittels des erfindungsgemäßen Systems das Erkennen des Zubehörartikels erreicht und dank der bereitgestellten Illuminationssignale leicht kontrollierbar. Darüber hinaus kann das System so eingerichtet sein, dass es eine Therapie oder ein Medikament vorschlagen kann, bspw. auch basierend auf einer Erkennung eines Zubehörartikels, wenn dieser und/oder die erfindungsgemäße Vorrichtung dafür eingerichtet und vorgesehen ist.

In einer vorteilhaften und bevorzugten Ausführungsform des medizinischen Systems nach der vorliegenden Erfindung ist die Steuereinheit dafür vorgesehen und angepasst, anhand des detektierten und damit identifizierten Zubehörartikels bei einer Einstellung einer Therapie der medizinischen Vorrichtungen mit vorbestimmter Kombination von dem Medikament oder Medikamentenkonzentration zu Zubehörartikel, einen optischen Alarm auszugeben und oder einen Start der Therapie zu unterbinden. Dabei kann die Bereitstellung des Illuminationssignals durch das Illuminationsmittel auf unterschiedliche Weise ausgestaltet werden, um einen derartigen Alarm als optisches Alarmsignal bereitzustellen, oder ggf. ein akustisches Alarmsignal optisch zu unterstützen. Beispielsweise kann der Alarm durch ein sich über die Zeit Verändern der Illuminationsintensität, insbesondere ein Blinken oder Pulsieren, dargestellt werden. Auch zusätzlich oder alternativ eine Veränderung der Farbe umfasst sein. Von der Erfindung umfasst sind auch Illuminationssignale, die Muster aus wechselnden Farben darstellen. Zur besseren Zuordnung kann eine medizinische Vorrichtung nach der vorliegenden Erfindung ein Anzeigeelement aufweisen, bspw. auf einem Benutzerinterface, aufweisen, zum Beispiel eine separate Leuchtanzeige, das ganze Display oder Teile des auf dem Display angezeigten Benutzerinterface, das eingerichtet ist, ein Signal bereitzustellen, das in der gleichen Farbe wie der Zubehörartikel, ein Marker des Zubehörartikels und/oder das Illuminationssignal gestaltet ist.

Das System kann weiter dafür eingerichtet sein, verschiedene Illuminationssignale, insbesondere verschiedene Farben, jeweils unterschiedlichen Funktionen des Systems und/oder der medizinischen Vorrichtung zuzuweisen. So kann beispielsweise ein optischer Alarm oder Hinweis farblich, zum Beispiel der Alarm mit roter Farbe, der Hinweis in oranger Farbe, oder mit wechselnder Blinkfrequenz oder statisch durch Illuminierung der Schlauchleitung visualisiert werden. Auch kann das Illuminationssignal ein Bestätigungssignal sein, bspw. um anzuzeigen, dass der gewünschte Einmalartikel mit dem gewünschten Medikament eingelegt wurde, oder dass der gewünschte Einmalartikel richtig eingelegt und angeschlossen wurde. Dies kann zum Beispiel durch eine passende Signalfarbe, zum Beispiel grün, oder durch ein passendes anderes Illuminationssignal, zum Beispiel eine gleichbleibende Lichtintensität, visualisiert werden.

So kann das Illuminationssignal ein Auftreten von speziellen Systemzuständen visualisieren und für den Benutzer unterscheidbar machen. Auch ein Alarm kann durch unterschiedliche Illuminationssignale visualisiert werden, ggf. auch, können unterschiedliche, den Alarm hervorrufende und detektierte Ursachen unterschieden werden. Diese umfassen Okklusions-Alarm, Luftalarm, Druckalarm, Alarm wegen eines falsch eingesetzten Zubehörartikels, Alarm wegen eines falschem aber korrekt eingesetzten Zubehörartikels, sowie Funktionsbeeinträchtigungen aller Art, ohne jedoch darauf beschränkt zu sein. Dies kann zu einer schnelleren, ergonomischen Fehlersuche und Fehlerbehandlung durch den Anwender vorteilhaft beitragen. Weiterhin kann das Aufheben und/oder das Quittieren eines Alarms ebenfalls zu einer Veränderung des Illumneszenzsignals führen, bspw. zum Farbwechsel oder Änderung der Intensität.

In einer vorteilhaften und bevorzugten Ausführungsform des medizinischen Systems nach der vorliegenden Erfindung, ist die Steuereinheit dafür vorgesehen und angepasst, anhand eines Markers des detektierten und damit identifizierten Zubehörartikels einen Teil einer vom Zubehörartikel umfassten und/oder angeschlossenen Schlauchleitung in einem Sichtbereich zu Illuminieren.

Insbesondere kann in einer Ausführungsform der vorliegenden Erfindung, die Farbe des Illuminationssignals abhängig von Informationen in der Medikation im Abgleich und/oder nach Abruf von Daten aus der Medikamentendatenbank gesteuert werden. In einer vorteilhaften und bevorzugten Ausführungsform des medizinischen Systems nach der vorliegenden Erfindung, ist die Steuereinheit dafür vorgesehen und angepasst, eine farbliche Illuminierung abhängig von einer Medikamentenauswahl in der Medikamentendatenbank zu hinterlegen und/oder einen Teil einer vom Zubehörartikel umfassten und/oder angeschlossenen Schlauchleitung in einem Sichtbereich abhängig von einer Medikamentenauswahl farbliche zu illuminieren und/oder an einen erkannten und zugeordneten Einmalartikel eine farbliche, dem Einmalartikel zugeordnete, Illuminierung abzugeben. Dabei kann ein bestimmtes, vorzugsweise eindeutiges, Illuminationssignal einer Medikamenten- und/oder Therapieart zugewiesen sein, oder einer anderen Art von Medikamentenkategorie, wie bspw. der Kritikalität des Medikaments und/oder der Therapie. Insbesondere kann sodann anhand der Erkennung und Zuordnung des Einmalartikels eine farbliche Illuminierung abgegeben werden, so dass anhand dieser, die eindeutige Zuordnung unmittelbar ersichtlich ist. Alternativ oder zusätzlich kann die Einstellung des Illuminationssignals über eine Bedienoberfläche der medizinischen Vorrichtung erfolgen.

Das erfindungsgemäße System kann durch im Stand der Technik vorhandene technische Möglichkeiten fortgebildet werden. Insbesondere kann die erfindungsgemäße Vorrichtung und/oder das erfindungsgemäße System ein Kommunikationsmodul enthalten, das bspw. als Drahtloskommunikationsmodul, insb. Wifimodul, ausgebildet ist. Hierdurch kann das System und/oder die Vorrichtung mit Netzwerken und Servern, einer Cloud oder dem Internet in Verbindung stehen und Daten, Steuersignale und Teile der Medikamentendatenbank austauschen, bereitstellen und/oder speichern. So kann ein erfindungsgemäßes System dazu eingerichtet sein eine Vielzahl erfindungsgemäßer Vorrichtungen aufeinander abgestimmt und/oder gleichzeitig zu betreiben, um einen einzelnen oder mehrere Patienten zu versorgen.

Von der Erfindung ebenfalls umfasst ist die Bereitstellung einer Illuminationsquelle an einer anderen Stelle des erfindungsgemäßen Zubehörartikels und/oder der erfindungsgemäßen medizinischen Vorrichtung. Insbesondere kann die Illuminationsquelle in ein an das System angeschlossenes weiteres Gerät angebracht und/oder angeschlossen sein. Zum Beispiel kann eine Lichtquelle so eingerichtet sein, dass sie manuell mit der Schlauchleitung lösbar verbunden werden kann, um das Illuminationssignal nur dann bereitzustellen, wenn das Illuminationssignal benötigt wird. Alternativ kann, wie hierin beschrieben, die Illuminationsquelle integraler Bestandteil der Schlauchleitung sein, und zum Beispiel, als Beschichtung der Schlauchleitung mit organischen Leuchtdioden oder gedruckten, elektrochemische Energiequellen ausgestaltet sein.

Die der Erfindung zu Grunde liegenden Aufgaben werden in einem vierten Aspekt gelöst durch ein medizinisches Verfahren zur optimierten Bedienung einer medizinischen Vorrichtung. Das Verfahren umfasst wenigstens die folgenden Schritte: einen Schritt a) des Einsetzens oder Anschließens eines Zubehörartikels in oder an eine medizinische Vorrichtung;
optional, einen Schritt des Erkennens eines Zubehörartikels anhand eines Markers des Zubehörartikels mittels einer Detektionseinheit der medizinischen Vorrichtung zur Detektion eines Zubehörartikels;
einen Schritt b) des Illuminierens eines Teiles einer vom Zubehörartikel umfassten und/oder angeschlossenen Schlauchleitung in einem Sichtbereich.

Es sei darauf hingewiesen, dass die oben angegebenen Schritte nicht zwangsläufig in der genannten Reihenfolge durchgeführt werden müssen. Die bereitgestellten Schritte können in jeder anderen geeigneten Reihenfolge durchgeführt werden. Die oben angegebene Reihenfolge kann jedoch für bestimmte Varianten des Verfahrens gelten. Zum Beispiel kann das Einsetzen oder Anschließen eines Zubehörartikels in oder an eine medizinische Vorrichtung vor oder nach dem Illuminieren eines Teiles einer vom Zubehörartikel umfassten und/oder angeschlossenen Schlauchleitung in einem Sichtbereich erfolgen. Im ersten Fall kann das Illuminieren vorteilhaft der Bestätigung und Orientierung des medizinischen Personals dienen, im zweiten Fall kann das Illuminieren dem medizinischen Personal helfen anhand der Illuminierung den gewünschten Zubehörartikel in oder an eine medizinische Vorrichtung einzusetzen oder anzuschließen.

Die der Erfindung zu Grunde liegenden Aufgaben werden in einem fünften Aspekt gelöst durch ein computerlesbares Speichermedium, umfassend Befehle, die bei einer Ausführung durch einen Computer diesen, Veranlassen, die Verfahrensschritte gemäß des Verfahrens nach dem vierten Aspekt auszuführen.

Alle beschriebenen Ausführungsformen der Erfindung haben den Vorteil, dass das bereitgestellte Illuminationssignal die Übersicht und damit Kontrolle vereinfacht, die erfindungsgemäße Vorrichtung zu betreiben und, insbesondere ein medizinisches Zubehörteil bzw. das erfindungsgemäße medizinische Zubehörteil, in die erfindungsgemäße Vorrichtung einzusetzen. Die vorliegende Erfindung in ihren verschiedenen Aspekten und Ausführungsformen reduziert vorteilhaft die Fehleranfälligkeit bei der Verwendung medizinischer Vorrichtungen der erfindungsgegenständlichen Art durch eine bessere Übersicht und vermeidet so Fehler in der Behandlung des Patienten. Zudem wird der Aufwand zur Betreuung komplexer Medikationsregime, insbesondere Infusionsregime, erheblich reduziert.

### KURZE BESCHREIBUNG DER FIGUREN

Die vorliegende Erfindung wird im Folgenden genauer beschrieben, unter Bezugnahme auf die Zeichnungen, aus denen weitere Merkmale, Ausführungsformen und Vorteile entnommen werden können. In den Zeichnungen zeigt:

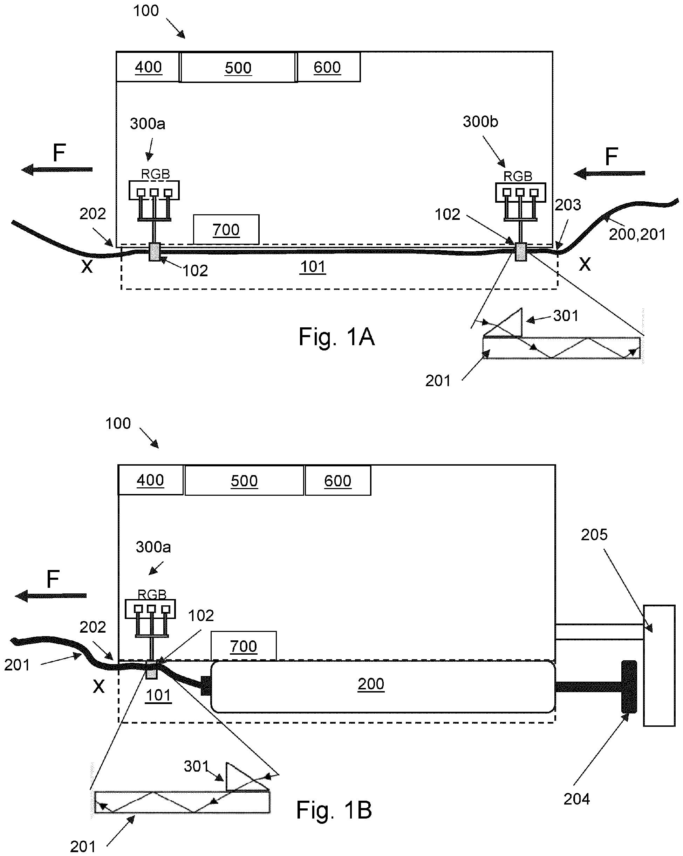

Fig. 1A eine schematische Darstellung einer ersten Ausführung einer erfindungsgemäßen medizinischen Vorrichtung nach dem ersten Aspekt, und
Fig. 1B eine schematische Darstellung einer zweiten Ausführung einer erfindungsgemäßen medizinischen Vorrichtung nach dem ersten Aspekt.

Die in der Beschreibung, den Ansprüchen, Beispielen und/oder den Figuren offenbarten Merkmale der vorliegenden Erfindung können sowohl einzeln als auch in beliebiger Kombination materiell für die Realisierung der Erfindung in verschiedenen Ausgestaltungen derselben sein.

### AUSFÜHRLICHE BESCHREIBUNG DER FIGUREN

In den in den Figuren gezeigten Ausführungsformen werden Elemente, die ähnliche oder identische Funktionen haben, mit gleichen Bezugszeichen bezeichnet. Es wird darauf hingewiesen, dass die Figuren möglicherweise nicht maßstabsgetreu zueinander sind.

Fig. 1A zeigt eine medizinische Vorrichtung 100 in Form einer volumetrischen Pumpe 100. Fig. 1B zeigt eine medizinische Vorrichtung 100 in Form einer Spritzenpumpe 100.

Die medizinische Vorrichtung umfasst eine Aufnahme oder einen Anschluss 101, hier mit gestrichelter Linie dargestellt, die eingerichtet ist, um einen medizinischen Zubehörartikel 200 einzusetzen.

Im Falle der volumetrischen Pumpe 100 in Fig. 1A, ist der Zubehörartikel 200 in der Form eines, nicht vollständig dargestellten Infusionsbeutelsystems 200 bereitgestellt, welches eine Schlauchleitung 201 aufweist, die in die Vorrichtung 100 eingesetzt ist. Durch die Schlauchleitung 201 wird in Richtung F kontrolliert eine medizinische Lösung und/oder ein Medikament mittels einer nicht dargestellten Schlauchpumpsystem der volumetrischen Pumpe 100 geführt. Derartige Pumpsysteme sind dem Fachmann im Stand der Technik bekannt. Im Falle, der in Fig. 1B dargestellten Spritzenpumpe 100 ist, der Zubehörartikel 200 in der Form einer Spritze 200 bereitgestellt, an welche eine Schlauchleitung 201 angebracht ist. Durch die Schlauchleitung 201 wird in Richtung F kontrolliert eine medizinische Lösung und/oder ein Medikament geführt, indem der Spritzenkolben 204 kontrolliert, vom Aktuator 205 der Spritzenpumpe 100 in das mit Medikament und/oder medizinischer Lösung befüllte Spritzenvolumen geführt wird, und sodann Medikament und/oder medizinische Lösung in die Schlauchleitung 201 abgegeben wird.

In beiden Fällen, sowohl in der in Fig. 1A als auch in der in Fig. 1B dargestellten Ausführungsform, umfasst die medizinische Vorrichtung 100 wenigstens eine Illuminationsquelle 300, insbesondere 300a, bzw. 300b, zum Illuminieren des eines Teiles einer vom Zubehörartikel 200 umfassten und/oder angeschlossenen Schlauchleitung 201, die in einem Sichtbereich x die Schlauchleitung 201 illuminiert. Im vorliegenden Fall ist als Illuminationsquelle 300, eine Lichtquelle 300 in Form von RGB-LEDs gewählt. Diese RGB-LED Anordnung 300 ist dazu eingerichtet, Licht verschiedener Wellenlängen zu erzeugen und somit den Sichtbereich x der Schlauchleitung 201 farbig zu illuminieren.

Die medizinische Vorrichtung 100 weist eine Führung 102 für die Schlauchleitung 201 auf, um die Schlauchleitung 201 in der Aufnahme oder dem Anschluss 101 zu fixieren. Diese Führung 102 umfasst dabei eine erste Illuminationsquelle 300a, die im Bereich der Führung 102 der Schlauchleitung 201 innerhalb der medizinischen Vorrichtung 100 so angeordnet ist, dass der Sichtbereich x der Schlauchleitung 201 im Bereich eines Austritts 202 der Schlauchleitung 201 aus der medizinischen Vorrichtung 100 beleuchtet wird, um die Schlauchleitung 201 in einem Sichtbereich x außerhalb der medizinischen Vorrichtung 100 zu illuminieren. Im Falle, der in Fig. 1A gezeigten volumetrischen Pumpe 100 ist weiter eine zweite Illuminationsquelle 300b vorgesehen, die ebenfalls im Bereich der Führung 102 der Schlauchleitung 201 und innerhalb der medizinischen Vorrichtung 100 angeordnet ist. In diesem Fall so, dass der Sichtbereich x der Schlauchleitung 201 im Bereich eines Eintritts 203 der Schlauchleitung 201 in die medizinische Vorrichtung 100 beleuchtet wird, um wiederum die Schlauchleitung 201 in einem Sichtbereich x außerhalb der medizinischen Vorrichtung 100 zu illuminieren. Dabei wird der Fachmann unmittelbar anerkennen, dass es im Falle, der in Fig. 1A gezeigten volumetrischen Pumpe 100 die Schlauchleitung 201 derart angeordnet ist, dass sie die Aufnahme 101 vollständig durchläuft und somit zwei Sichtbereiche x, nämlich einmal am Eintritt 203 und einmal am Austritt 202, aufweist. Beide Sichtbereiche werden je-weils durch die Lichtquelle 300a bzw. 300b illuminiert.

Schematisch in Vergrößerung dargestellt ist jeweils die Führung 102 für die Schlauchleitung 201. Diese weist ein Prisma 301 als Lichtweiche 301 auf, um das durch die RGB LEDs 300a und 300b jeweils bereitgestellte Lichtsignal in die Schlauchleitung 201 einzukoppeln. Der Gang der Lichtstrahlen ist schematisch durch aufeinanderfolgende Pfeile dargestellt und es ist ersichtlich, dass das in die Schlauchleitung 201 eingekoppelte Lichtsignal innerhalb der Schlauchleitung 201, bevorzugt in einem Mantelteil der Schlauchleitung 201, geführt wird. Je nach Beschaffenheit des Materials, kann das Lichtsignal weiter oder weniger weit in der Schlauchleitung 201 vordringen und der Sichtbereich x wird somit über eine längere oder kürzere Strecke illuminiert werden.

Die dargestellten medizinischen Zubehörartikel 200 sind insbesondere Einmalartikel 200, die üblicherweise aus einem lichtdurchlässigen, flüssigkeitsdichten und elastischen Kunststoffmaterial gefertigt sind. Wenigstens im Abschnitt der Lichteinkopplung 301 weisen diese einen Hohlkörper in Form einer Schlauchleitung 201 auf und lassen sich in die medizinische Vorrichtung 100 einsetzen und in die Führung(en) 102 einbringen. Ein solcher medizinischer Zubehörartikel 200 kann einen Lichtwellenleiter und/oder ein Lichtreflektionsmittel umfassen und insbesondere auch eine eigene (hier nicht dargestellte) Illuminationsquelle 300c, hier nicht gezeigt, zum Illuminieren eines Teiles einer vom Zubehörartikel 200 umfassten und/oder angeschlossenen Schlauchleitung 201 im Sichtbereich x.

Ein durch die vorliegende Erfindung bereitgestelltes medizinisches System umfasst wenigstens eine medizinische Vorrichtung 100 und wenigstens ein Zubehörteil 200, sowie eine Speichereinheit 400 mit einer Medikamentendatenbank, in welcher Daten zu Medikamenten hinterlegt sind und eine in der Speichereinheit 400 gespeicherte Zubehörartikel-Datenbank, in welcher Daten zu zumindest zwei unter-schiedlichen Zubehörartikeln 200 hinterlegt sind. Eine Detektionseinheit 700 ist vorgesehen zum Erkennen und Klassifizieren eines Zubehörartikels anhand eines Markers des Zubehörartikels mittels eines Sensors der Detektionseinheit 700. Steuereinheit 500 ist vorgesehen und angepasst, anhand eines detektierten und damit identifizierten Zubehörartikels 200, einen Teil einer vom Zubehörartikel 200 umfassten und/oder angeschlossenen Schlauchleitung 201 in einem Sichtbereich x zu Illuminieren. In einem solchen erfindungsgemäßen System kann die Steuereinheit 500 dafür vorgesehen und angepasst sein, anhand des detektierten und damit identifizierten Zubehörartikels 200 bei einer Einstellung einer Therapie der medizinischen Vorrichtung 100 mit vorbestimmter Kombination von dem Medikament oder Medikamentenkonzentration zu Zubehörartikel 200, einen optischen Alarm auszugeben und oder einen Start der Therapie zu unterbinden. Weist der Zubehörartikels 200 einen Marker auf, kann die Steuereinheit 500 dafür vorgesehen und angepasst sein, anhand des Markers des detektierten und damit identifizierten Zubehörartikels 200 einen Teil einer vom Zubehörartikel 200 umfassten und/oder angeschlossenen Schlauchleitung 201 in einem Sichtbereich x zu Illuminieren. Insbesondere kann die Steuereinheit 500 dafür vorgesehen und angepasst sein, eine farbliche Illuminierung abhängig von einer Medikamentenauswahl in der Medikamentendatenbank zu hinterlegen und/oder einen Teil einer vom Zubehörartikel 200 umfassten und/oder angeschlossenen Schlauchleitung 201 in einem Sichtbereich (x) abhängig von einer Medikamentenauswahl farbliche zu illuminieren.

Mit den gezeigten erfindungsgemäßen Ausführungsformen kann insbesondere ein erfindungsgemäßes medizinisches Verfahren zur optimierten Bedienung einer medizinischen Vorrichtung 100, umfassend wenigstens die folgenden Schritte, durchgeführt werden:
einen Schritt a) des Einsetzens oder Anschließens eines Zubehörartikels 200 in oder an eine medizinische Vorrichtung 100;
einen Schritt b) des Illuminierens eines Teiles einer vom Zubehörartikel 200 umfassten und/oder angeschlossenen Schlauchleitung 201 in einem Sichtbereich (x).

Ein computerlesbares Speichermedium 600 kann Befehle umfassen, die bei einer Ausführung durch einen Computer diesen veranlassen, die Verfahrensschritte des erfindungsgemäßen Verfahrens auszuführen und/oder zu kontrollieren.

Die in den Figuren gezeigten Ausführungsformen können sich auf bevorzugte Ausführungsformen beziehen, während alle Elemente und Merkmale, die in Verbindung mit den Ausführungsformen beschrieben werden, sofern angebracht, in Kombination mit jeder anderen Ausführungsform und jedem anderen Merkmal verwendet werden können, wie hierin beschrieben, insbesondere in Bezug auf jede andere Ausführungsform, die weiter oben beschrieben wurde.

### Bezugszeichenliste

- 100: Medizinische Vorrichtung
- 101: Aufnahme oder einen Anschluss
- 102: Führung für Schlauchleitung
- 200: medizinischer Zubehörartikel
- 201: Schlauchleitung
- 202: Austritt
- 203: Eintritt
- 204: Spritzenkolben
- 205: Aktuator
- 300: Illuminationsquelle
- 301: Lichtweiche, Prisma
- 400: Speichereinheit
- 500: Steuereinheit
- 600: computerlesbares Speichermedium
- 700: Sensor, Detektionseinheit

- X: Sichtbereich
- F: Flussrichtung

## Patentansprüche

1. Medizinische Vorrichtung (100), insbesondere eine medizinische Pumpe, umfassend eine Aufnahme oder einen Anschluss (101), eingerichtet zum Einsetzen eines medizinischen Zubehörartikels (200), insbesondere eines medizinischen Einmalartikels, umfassend eine oder verbindbar mit einer Schlauchleitung (201), umfassend
eine Illuminationsquelle (300) zum Illuminieren des eines Teiles einer vom Zubehörartikel (200) umfassten und/oder angeschlossenen Schlauchleitung (201) in einem Sichtbereich (x).

2. Medizinische Vorrichtung (100) nach Anspruch 1, wobei die Illuminationsquelle (300) eine Lichtquelle (300) ist, die bevorzugterweise ausgewählt ist aus der Gruppe umfassend LED und Laser.

3. Medizinische Vorrichtung (100) nach Anspruch 1 oder 2, wobei die medizinische Vorrichtung (100) eine Führung (102) für die Schlauchleitung (201) umfasst, und wobei
- die Illuminationsquelle (300) eine erste Illuminationsquelle (300) ist, die im Bereich der Führung (102) der Schlauchleitung (201) innerhalb der medizinischen Vorrichtung (100) so angeordnet ist, dass der Sichtbereich (x) der Schlauchleitung (201) im Bereich eines Austritts (202) der Schlauchleitung (201) aus der medizinischen Vorrichtung (100) beleuchtet wird, um die Schlauchleitung (201) in einem Sichtbereich (x) außerhalb der medizinischen Vorrichtung (100) zu illuminieren und/oder wobei
- die Illuminationsquelle (300) eine zweite Illuminationsquelle (300) ist, die im Bereich der Führung (102) der Schlauchleitung (201) innerhalb der medizinischen Vorrichtung (100) so angeordnet ist, dass der Sichtbereich (x) der Schlauchleitung (201) im Bereich eines Eintritts (203) der Schlauchleitung (201) in die medizinische Vorrichtung (100) beleuchtet wird, um die Schlauchleitung (201) in einem Sichtbereich (x) außerhalb der medizinischen Vorrichtung (100) zu illuminieren.

4. Medizinische Vorrichtung (100) nach einem der Ansprüche 1 bis 3, wobei die Illuminationsquelle (300) eine oder mehrere Lichtquellen (300) umfasst, und die bevorzugt dazu eingerichtet ist, Licht verschiedener Wellenlängen zu erzeugen.

5. Medizinische Vorrichtung (100) nach einem der Ansprüche 1 bis 4, wobei die medizinische Vorrichtung (100) eine Detektionseinheit (700) mit einem Sensor zur Detektion des Zubehörartikels (200) umfasst und/oder zur Klassifizierung und/oder Einordnung des Zubehörartikels (200) umfasst.

6. Medizinische Vorrichtung (100) nach einem der Ansprüche 1 bis 5, wobei die medizinische Vorrichtung (100) eine Lichtweiche (301), insbesondere ein Prisma, umfasst.

7. Medizinischer Zubehörartikel (200), insbesondere Einmalartikel, wobei der Zubehörartikel (200) wenigstens abschnittsweise einen Hohlkörper aufweist und zum Einsetzen in eine Medizinische Vorrichtung (100) eingerichtet ist, wobei bevorzugt die medizinische Vorrichtung (100) eine medizinische Vorrichtung (100) nach einem der Ansprüche 1 bis 7 ist.

8. Medizinischer Zubehörartikel (200) nach Anspruch 7, umfassend einen Lichtwellenleiter und/oder ein Lichtreflektionsmittel.

9. Medizinischer Zubehörartikel (200) nach Anspruch 7 oder 8, umfassend eine Illuminationsquelle (300) zum Illuminieren eines Teiles einer vom Zubehörartikel (200) umfassten und/oder angeschlossenen Schlauchleitung (201) in einem Sicht-bereich (x).

10. Medizinisches System umfassend wenigstens eine medizinische Vorrichtung (100), bevorzugt nach einem der Ansprüche 1 bis 6, und wenigstens ein Zubehörteil (200), bevorzugt nach einem der Ansprüche 7 bis 9, weiter umfassend
- eine Speichereinheit (400) mit einer Medikamentendatenbank, in welcher Daten zu Medikamenten hinterlegt sind und/oder vorzugsweise einer in der Speichereinheit (400) gespeicherten Zubehörartikel-Datenbank, in welcher Daten zu zumindest zwei unterschiedlichen Zubehörartikel (200) hinterlegt sind, und
- eine Steuereinheit (500), die dafür vorgesehen und angepasst ist, an-hand des detektierten und damit identifizierten Zubehörartikels (200), einen Teil einer vom Zubehörartikel (200) umfassten und/oder angeschlossenen Schlauchleitung (201) in einem Sichtbereich (x) zu illuminieren.

11. Medizinisches System nach Anspruch 10, wobei die Steuereinheit (500) dafür vorgesehen und angepasst ist, anhand des detektierten und damit identifizierten Zubehörartikels (200) bei einer Einstellung einer Therapie der medizinischen Vorrichtung (100) mit vorbestimmter Kombination von dem Medikament oder Medikamentenkonzentration zu Zubehörartikel (200), einen optischen Alarm auszugeben und/oder einen Start der Therapie zu unterbinden.

12. Medizinisches System nach Anspruch 10 oder 11, wobei die Steuereinheit (500) dafür vorgesehen und angepasst ist, anhand eines Markers des detektierten und damit identifizierten Zubehörartikels (200) einen Teil einer vom Zubehörartikel (200) umfassten und/oder angeschlossenen Schlauchleitung (201) in einem Sichtbereich (x) zu Illuminieren.

13. Medizinisches System nach einem der Ansprüche 10 bis 12, wobei die Steuereinheit (500) dafür vorgesehen und angepasst ist,
- eine farbliche Illuminierung abhängig von einer Medikamentenauswahl in der Medikamentendatenbank zu hinterlegen und/oder
- einen Teil einer vom Zubehörartikel (200) umfassten und/oder angeschlossenen Schlauchleitung (201) in einem Sichtbereich (x) abhängig von einer Medikamentenauswahl farbliche zu illuminieren und/oder
- an einen erkannten und zugeordneten Einmalartikel eine farbliche, dem Einmalartikel zugeordnete, Illuminierung abzugeben.

14. Medizinisches Verfahren zur optimierten Bedienung einer medizinischen Vorrichtung, umfassend wenigstens die folgenden Schritte:
einen Schritt a) des Einsetzens oder Anschließens eines Zubehörartikels (200) in oder an eine medizinische Vorrichtung (100);
optional, einen Schritt des Erkennens eines Zubehörartikels anhand eines Markers des Zubehörartikels mittels einer Detektionseinheit (700) der medizinischen Vorrichtung zur Detektion eines Zubehörartikels;
einen Schritt b) des Illuminierens eines Teiles einer vom Zubehörartikel (200) umfassten und/oder angeschlossenen Schlauchleitung (201) in einem Sichtbereich (x).

15. Computerlesbares Speichermedium (600), umfassend Befehle, die bei einer Ausführung durch einen Computer diesen veranlassen, die Verfahrensschritte gemäß Anspruch 14 auszuführen und/oder zu kontrollieren.
